# EUROPÄISCHE PATENTANMELDUNG

(11) **EP 1 010 700 A1**
(43) Veröffentlichungstag der Anmeldung: **21.06.2000**
(21) Anmeldenummer: 99121190.5
(22) Anmeldetag: 22.10.1999
(51) Int. Cl.: C07D 473/18

(54) **Verfahren zur Herstellung von Guanin unter Druck**

(30) Priorität: 16.12.1998 DE 19857949
(71) Anmelder: Degussa-Hüls Aktiengesellschaft, 60287 Frankfurt am Main (DE)
(72) Erfinder: Theis, Christoph, Dr., 53859 Niederkassel (DE); Bruhn, Stefan, Dr., 21447 Handorf (DE)

(57) **Zusammenfassung**

Die vorliegende Erfindung betrifft ein Verfahren zur Herstellung von Guanin, wobei 2,4-Diamino-5-formylamino-6-hydroxypyrimidin (DAFHP) in konzentrierter Ameisensäure in Abwesenheit von Formamid ggf. in Anwesenheit katalytischer Mengen eines reduzierend wirkenden Additivs bei erhöhter Temperatur und erhöhtem Druck umgesetzt wird.

## Beschreibung

Die vorliegende Erfindung betrifft ein Verfahren zur Herstellung von Guanin (2-Amino-1,9-dihydropurin-6-on) aus 2,4-Diamino-5-formylamino-6-hydroxypyrimidin (DAFHP) unter Druck.

Die Nucleinsäurebase Guanin ist als wichtiges Zwischenprodukt zur Synthese pharmakologisch aktiver Verbindungen, insbesondere antiviraler Wirkstoffe, von großer Bedeutung. Guanin wird z. B. als Vorstüfe für Acyclovir, das gemäß DE-A-35 44 461 zur Therapie von Virusinfektionen geeignet ist, benötigt.

Die Umsetzung von 4,5-Diaminopyrimidin-Sulfaten mit Formamid zu den entsprechenden Purinen ist literaturbekannt (Robins et al., J. Am. Chem. Soc. 75 (1953) 263). Zur Synthese des Guanins wird 2,4,5-Triamino-6-hydroxypyrimidin-Sulfat (TAHP-sulfat) eingesetzt.

Nach der DE-A-37 29 471 kann Guanin durch Erhitzen einer Suspension von TAHP-Sulfat in Formamid bis zu 200 °C unter Abdestillation des gebildeten Reaktionswassers erhalten werden.

Nachteilig ist die Eigenschaft des Formamids, sich bei den benötigten hohen Temperaturen teilweise zu zersetzen, was neben der Bildung von Kohlenmonoxid und Ammoniak verfärbte Guanin-Rohprodukte zur Folge hat, die einen hohen Reinigungsaufwand erfordern. Die Notwendigkeit, das in freier Form unbeständige TAHP in Form seines Sulfats einzusetzen, bedingt eine hohe Salzfracht, was einen weiteren Nachteil darstellt.

Gemäß der EP-A-0 415 028 wird Guanin durch Umsetzung von TAHP-Sulfat mit Alkaliformiat und Ameisensäure erhalten. Dieses Verfahren vermeidet zwar die mit dem Einsatz von Formamid verbundenen Nachteile, führt jedoch ebenfalls zu einem wirtschaftlich und ökologisch ungünstigen hohen Zwangsanfall an Salzfracht in Form von Alkalisulfat in der Reaktionsmischung.

Bei den genannten, auf TAHP-Sulfat basierenden Verfahren wird als Zwischenprodukt 2,4-Diamino-5 -formylamino-6-hydroxypyrimidin (im folgenden als DAFHP bezeichnet) durchlaufen, das in situ zum Guanin umgesetzt wird.

Nach der DE-A-41 36 114 kann Guanin auch ausgehend von isoliertem DAFHP durch Erhitzen in Formamid auf mindestens 140 °C gewonnen werden. Das Verhältnis von DAFHP zu Formamid beträgt 1:2 bis 1:3. Der Reaktionsmischung kann bis zu 10 % Ameisensäure zugesetzt werden. Das dabei eingesetzte DAFHP wird beispielsweise durch ein Verfahren nach der EP-A-0 267 594 erhalten, worin 2,4-Diamino-6-hydroxy-5-nitrosopyrimidin katalytisch hydriert und zu TAHP-Sulfat umgesetzt wird. Nach der Hydrierung wird das Reaktionsgemisch mit Ameisensäure, gegebenenfalls unter Zusatz einer Mineralsäure, versetzt, um DAFHP in hohen Ausbeuten zu erhalten. Das in der DE-A-41 36 114 beschriebene Verfahren ist zwar salzfrei, weist allerdings die bereits erwähnten Nachteile auf, die mit der Verwendung von Formamid (wie Zersetzung, aufwendige Reinigung des Endprodukts) einhergehen. Das nach der DE-A-41 36 114 gewonnene Guanin wird mit einem Gehalt (HPLC) von weniger als 98,0 % erhalten. Nach der Reinigung kommt es daher zu Ausbeuteverlusten.

Nach der deutschen Anmeldung P 198 39 013.0 kann Guanin auch ausgehend von isoliertem DAFHP durch Erhitzen in siedender Ameisensäure, die ggf mit geringen Wasserzusätzen verdünnt sein kann, hergestellt werden. Bei diesem Verfahren sind lange Reaktionszeiten von bis zu 20 Stunden erforderlich.

Allen vorgenannten Verfahren ist neben den beschriebenen verfahrenstechnischen Nachteilen gemein, daß hierbei Produkte resultieren, die auch nach ein- oder mehrmaliger Umfällung mit A-Kohle z. T. erhebliche Konzentrationen einer durch Dünnschicht-Chromatographie nachweisbaren, bei 366 nm fluoreszierenden Nebenkomponente enthalten.

Es ist daher die Aufgabe der vorliegenden Erfindung, ein technisches Verfahren zur Herstellung von Guanin bereitzustellen, welches die genannten Nachteile des Standes der Technik nicht aufweist und bei dem Guanin in hoher Raum-Zeit-Ausbeute und extrem hoher Reinheit erhalten wird.

Diese Aufgabe wird erfindungsgemäß durch ein Verfahren zur Herstellung von Guanin ausgehend von 2,4-Diamino-5-formylamino-6-hydroxypyrimidin (DAFHP) gelöst, das dadurch gekennzeichnet ist, daß man isoliertes DAFHP in konzentrierter Ameisensäure einer Cyclokondensation unter Druck bei Temperaturen oberhalb des Siedepunktes der Ameisensäure unterwirft und diese Reaktion ggf. in Anwesenheit katalytischer Mengen eines reduzierend wirkenden Additivs durchführt.
Das hierbei zum Einsatz gelangende isolierte DAFHP ist beispielsweise durch das in der EP-A-0 267 594 beschriebene Verfahren leicht zugänglich.

Es war bei der erfindungsgemäßen Verfahrensweise nicht ohne weiteres zu erwarten, daß eine geringere Temperatur-Erhöhung im Vergleich zu den in der deutschen Anmeldung P 198 39 013.0 genannten Temperaturen bei Verwendung von reiner Ameisensäure unter Überdruck eine praktisch vollständige Cyclokondensation des eingesetzten DAFHP in überraschend kurzen Reaktionszeiten ermöglicht und hierbei nach Aufarbeitung hochreines Guanin erhalten wird.

Als sehr überraschend hat sich ferner erwiesen, daß durch Zusatz von reduzierend wirkenden Additiven die Reinheit des so erhaltenen Guanins, insbesondere im Hinblick auf fluoreszierende Nebenkomponenten, signifikant verbessert werden kann und nach Aufarbeitung praktisch nebenproduktfreies Rein-Guanin erhalten wird.

Als reduzierend wirkende Additive gelangen bei dem hier beschriebenen Verfahren Alkali- oder Erdalkali-Salze von niedervalenten Oxo-Säuren des Schwefels zum Einsatz, sehr bevorzugt sind hierbei beispielsweise Natriumsulfit oder Natriumdithionit.

Bezogen auf eingesetztes DAFHP werden von den Salzen der niedervalenten Schwefel-Oxo-Säuren bis zu 7,5 Mol-% eingesetzt.

Desweiteren können auch reduzierend wirkende Edelmetalle, bevorzugt solche aus der 8. Nebengruppe des Periodensystems, die ggf. in metallischer Form auf Träger aufgebracht sind, zum Einsatz gelangen. Sehr bevorzugt ist bei dieser Verfahrensweise der Einsatz von metallischem Palladium oder Platin, insbesondere in Verbindung mit Kohle als Trägermaterial.

Von den auf Trägern fixierten Edelmetallen, die bis zu 50 % wasserfeucht sind und - bezogen auf die Katalysatortrockenmasse - bis zu 10 % Edelmetall enthalten können, werden bis zu 10 Gew. -%, bezogen auf eingesetztes DAFHP, eingesetzt.

Die wasserfeuchten Träger-Katalysatoren könnnen vor dem Einsatz beispielsweise durch Waschen mit konzentrierter Ameisensäure von anhaftendem Restwasser befreit und dann ameisensäurefeucht eingesetzt werden, jedoch bringt diese Verfahrensweise wegen des dadurch aufwendigeren Handlings keine signifikanten Vorteile.

Es hat sich vielmehr überraschend gezeigt, daß die zum Einsatz gelangenden Edelmetall-Träger-Katalysatoren als kommerzielle Produkte, d. h. wasserfeucht, eingesetzt werden können, wobei der Edelmetall-Gehalt, bezogen auf die Trockenmasse des Trägerkatalysators, bevorzugt bis zu 5 % beträgt.
Desweiteren ist es ohne weiteres möglich, den im Zuge der Rohprodukt-Reinigung isolierten Edelmetall/Träger-Katalysator ohne erkennbare Nachteile mehrfach für neue DAFHP-Cyclokondensationen einzusetzen, oder durch Zusatz geringer Mengen Frisch-Katalysator zu ergänzen.

In der bevorzugten Ausführungsform werden isoliertes DAFHP, reduzierend wirkendes Additiv und handelsübliche konzentrierte Ameisensäure gemischt und in einem geeigneten Druck-Reaktor auf Temperaturen von 110 bis 140 °C erhitzt, wobei Reaktionstemperaturen von 120 bis 130 °C bevorzugt zur Anwendung gelangen; die Reaktionszeiten betragen im allgemeinen 3 bis 8, bevorzugt 4 bis 6 Stunden.

Kürzere Reaktionszeiten haben deutliche Ausbeuteverluste durch nicht umgesetztes DAFHP zur Folge; längere Reaktionszeiten sind prinzipiell möglich, führen jedoch nicht zu signifikanten Verbesserungen. Zudem wiedersprechen sie dem erfindungsgemäßen Ziel einer hohen Raum-Zeit-Ausbeute.

Niedrigere Temperaturen verlängern die Reaktionszeiten infolge schleppenden DAFHP-Umsatzes, höhere Reaktionstemperaturen haben signifikant höhere Zersetzungsraten der eingesetzten Ameisensäure und somit wirtschaftliche Nachteile zur Folge.

Es hat sich hierbei überraschend gezeigt. daß das erfindungsgemäße Verfahren den Einatz hoher DAFHP-Konzentrationen bei sehr hohen Raum-Zeit-Ausbeuten erlaubt, was zweifellos einen besonderen Vorteil darstellt.

Die Molalität des Reaktionsgemisches (bezogen auf DAFHP) beträgt in der Ausführungsform 1,5 bis 2,8, bevorzugt 1,7 bis 2,2. Unter diesen Bedingungen sind die zum Einsatz gelangenden Reaktionsgemische sehr gut rührbar.

Im erfindungsgemäßen Verfahren wird beispielsweise im Vergleich zur EP-A-0 415 028 eine mehr als vierfache Raum-Zeit-Ausbeute erzielt.
Es ist im Verlauf der Cyclokondensation nicht erforderlich, den sich aufgrund der gewählten Reaktions-Temperatur und infolge geringer Ameisensäure-Zersetzung aufbauenden Druck durch geeignete technische Maßnahmen zu begrenzen, jedoch kann dies aus praktischen Erwägungen z. B. im Druckbereich 2-20 bar, bevorzugt 4 bis 6 bar, ohne Nachteile erfolgen.

In dem erfindungsgemäßen Verfahren werden nach beendeter Reaktion Ameisensäure und Wasser abdestilliert, was vorzugsweise unter vermindertem Druck erfolgt.
Die so zurückgewonnene wasserhaltige Ameisensäure ist sehr rein und kann in anderen Verfahren eingesetzt werden. Insbesondere kann die zurückgewonnene wasserhaltige Ameisensäure bei der Herstellung des Ausgangsstoffs DAFHP verwendet werden, was einen weiteren Vorteil des erfindungsgemäßen Verfahrens darstellt.

Die Reinigung des erhaltenen Roh-Guanins kann in bekannter Weise erfolgen, wobei die bevorzugte Ausführungsform ggf. auch die leichte Abtrennung von trägerhaltigen Edelmetall-Katalysatoren erlaubt.

Bei der sehr bevorzugten Verfahrensweise zur Reinigung des Roh-Guanins wird dieses in wäßrigem Alkalihydroxid gelöst, ggf. enthaltener Edelmetall-Träger-Katalysator abfiltriert und gewaschen, und das ggf. erhaltene Gesamt-Filtrat mit Aktivkohle behandelt. Anschließend wird Guanin üblicherweise durch Ausfällung, vorzugsweise durch Verseifungsfällung, entsprechend der DE-A-37 23 874, isoliert. Das erfindungsgemäße Verfahren liefert Roh-Guanin in sehr guten Ausbeuten von beispielsweise 98 % d. Th.

Nach Reinigung kann Guanin in guten Ausbeuten von üblicherweise 92 % d. Th. und mehr als extrem reines Endprodukt erhalten werden, wobei hierbei Gehalte (HPLC) von mehr als 99,8 % erzielt werden.

Dünnschichtchromatographische Untersuchungen (DC) dieser Rein-Guanine weisen im Vergleich zu einem nach der EP-A-0 415 028 hergestellten und durch zweimalige Reinigung mit jeweils 30 Gew.-% A-Kohle (bezogen auf eingesetztes Guanin) nebst Verseifungsfällungen nach DE-A-37 23 874 hergestellten Guanin keine erkennbaren fluoreszierenden Nebenkomponenten auf.

Das nach dem erfindungsgemäßen Verfahren hergestellte Guanin kann als Zwischenprodukt zur Synthese pharmakologisch wirksamer Verbindungen verwendet werden.

Die nachfolgenden Beispiele sollen die Erfindung näher erläutern, ohne sie jedoch auf diese zu beschränken.

### Vergleichsbeispiel: In einem mit Drehrührer ausgestatteten Glas-Autoklaven werden 333,3 g Ameisensäure (98-100 %ig) vorgelegt und unter Rühren portionsweise 101,4 g (0,6 mol) DAFHP eingetragen (bezogen auf DAFHP beträgt die Molalität 1,8), auf 120 °C erhitzt und die gut rührbare Suspension anschließend 6 Stunden bei dieser Temperatur belassen. Während der Reaktion wird der sich aufbauende Druck durch sukzessives Entspannen im Bereich von 4 bis 5 bar gehalten.

Nach Abkühlung wird die Suspension entnommen und Ameisensäure und Wasser werden im Wasserstrahlvakuum nahezu vollständig abdestilliert.
Das erhaltene Roh-Guanin wird in bekannter Weise durch Lösen in wäßrigem Alkalihydroxid, Behandeln mit A-Kohle (30 Gew.-%) und anschließende Verseifungsfällung gereinigt.
Aus dem erhaltenden Roh-Produkt werden so nach einmaliger Anwendung des Reinigungsverfahrens 84,0 g (92,6 % d. Th.) Guanin mit einem Gehalt (HPLC) von 99,8 % erhalten.
Der quantitative DC-Vergleich mit der beschriebenen, doppelt gereinigten Referenzcharge gemäß EP-A-0 415 028 weist gleiche Konzentrationen an fluoreszierenden Nebenkomponenten auf.

### Beispiel 1:

In 333,3 g Ameisensäure (98-100 %ig) werden unter Rühren portionsweise (0,6 mol) DAFHP (bezogen auf DAFHP beträgt die Molalität 1,8) eingetragen und mit 4,0 g Pd/C-Katalysator (50 % wasserfeucht, Pd-Gehalt: 5 %) versetzt.

Das Gemisch wird im Glas-Autoklaven bis 120 °C erhitzt und die gut rührbare Suspension 5 Stunden bei dieser Temperatur belassen, wobei der sich einstellende Druck durch sukzessives Entspannen im Bereich von 4 bis 5 bar gehalten wird.

Das nach Abkühlung und Entspannen entnommene Reaktionsgemisch wird durch Destillation im Wasserstrahlvakuum nahezu vollständig von Ameisensäure und Wasser befreit.

Das so erhaltene Roh-Guanin weist im HPLC keine Rest-DAFHP aus und wird in bekannter Weise durch Lösen in Alkalihydroxid, Behandeln mit A-Kohle (10 Gew.-%) und anschließende Verseifungsfällung gereinigt.
Aus dem erhaltenen Roh-Produkt werden so nach einmaliger Anwendung des Reinigungsverfahrens 85,2 g (94,0 % d. Th.) Guanin mit einem Gehalt von 99,9 % (nach HPLC) erhalten.
Dünnschichtchromatographisch lassen sich bei diesem erhaltenen Rein-Guanin keine fluoreszierenden Nebenkomponenten nachweisen.

### Beispiel 2:

Analog Beispiel 1, aber unter Verwendung von 3,8 g Natriumsulfit (5,0 Mol-%) und 5 Stunden bei 123 C°.

Das so erhaltene Reaktionsgemisch wird wie in Beispiel 1 aufgearbeitet und das so erhaltene Roh-Guanin (Rest-DAFHP-Gehalt nach HPLC: 0,3 %) wie in Beispiel 1 beschrieben unter Verwendung von 30 Gew.-% A-Kohle gereinigt.
Das so erhaltene Rein-Guanin (84,4 g entsprechend 93,1 % d. Th.) weist einen Gehalt von 99,8 % (HPLC) auf und weist im quantitativen DC-Vergleich mit der beschriebenen, doppelt gereinigten Referenzcharge nur eine extrem geringe Menge an fluoreszierenden Nebenkomponenten auf.

## Patentansprüche

1. Verfahren zur Herstellung von Guanin aus 2,4-Diamino-5-formylamino-6-hydroxypyrimidin,
dadurch gekennzeichnet,
daß man isoliertes 2,4-Diamino-5-formylamino-6-hydroxypyrimidin in Ameisensäure mit oder ohne Gegenwart von reduzierend wirkenden Additiven unter erhöhter Temperatur und erhöhtem Druck zum Guanin umsetzt.

2. Verfahren nach Anspruch 1,
dadurch gekennzeichnet,
daß als reduzierend wirkendes Additiv Alkali- oder Erdalkalisalze niedervalenter Oxosäuren des Schwefels eingesetzt werden.

3. Verfahren nach Anspruch 1,
dadurch gekennzeichnet,
daß Edelmetalle aus der 8. Nebengruppe des Periodensystems der Elemente als reduzierend wirkende Additive eingesetzt werden, die als Metall oder als Metall auf geeigneten Trägermaterialien vorliegen.

4. Verfahren nach Anspruch 3,
dadurch gekennzeichnet,
daß Palladium oder Platin als Edelmetall-Katalysator eingesetzt wird.

5. Verfahren nach einem der vorhergehenden Ansprüche,
dadurch gekennzeichnet,
daß Kohle als Edelmetall-Träger eingesetzt wird.

6. Verfahren nach einem der vorhergehenden Ansprüche,
dadurch gekennzeichnet,
daß die Reaktion bei Temperaturen von 110 bis 140 °C durchgeführt wird.

7. Verfahren nach einem der vorhergehenden Ansprüche,
dadurch gekennzeichnet,
daß die Reaktion bei mindestens dem Druck ausgeführt wird, der sich einstellt. wenn Ameisensäure unter Eigendruck auf Temperaturen von 110 bis 140 °C erhitzt wird.

8. Verfahren nach einem der vorhergehenden Ansprüche,
dadurch gekennzeichnet,
daß die Reaktionszeit 3 bis 8 Stunden, bevorzugt 4 bis 6 Stunden, beträgt.

9. Verfahren nach einem der vorhergehenden Ansprüche,
dadurch gekennzeichnet,
daß die molale Konzentration an 2,4-Diamino-5-formylamino-6-hydroxypyrimidin im Reaktionsgemisch 1,5 bis 2,8, bevorzugt 1,7 bis 2,2, beträgt.

10. Verfahren nach einem der vorhergehenden Ansprüche,
dadurch gekennzeichnet,
daß konzentrierte Ameisensäure eingesetzt wird.

11. Verfahren nach einem der vorhergehenden Ansprüche,
dadurch gekennzeichnet,
daß nach beendeter Reaktion Ameisensäure und Wasser unter vermindertem Druck abdestilliert werden und das so erhaltene Guanin in bekannter Weise in wäßrig alkalischer Lösung mit Aktivkohle gereinigt und nach anschließender Ausfällung, bevorzugt durch Verseifungsfällung, isoliert wird.

12. Verwendung des nach einem der Ansprüche 1 bis 11 hergestellten Guanins als Zwischenprodukt zur Synthese pharmakologisch wirksamer Verbindungen.
